# EUROPÄISCHE PATENTSCHRIFT

(11) **EP 0 759 896 B1**
(45) Veröffentlichungstag und Bekanntmachung des Hinweises auf die Patenterteilung: **30.01.2002**
(21) Anmeldenummer: 95920071.8
(22) Anmeldetag: 19.05.1995
(51) Int. Cl.: C07C 29/141, C07C 29/145, C07C 31/20, C07C 31/12, C07C 31/27, C07C 31/125

(54) **KATALYSATOR UND VERFAHREN ZUR HERSTELLUNG VON ALKOHOLEN**
ALCOHOL PREPARATION PROCESS AND CATALYST
PROCEDE ET CATALYSEUR POUR LA PREPARATION D'ALCOOLS

(30) Priorität: 19.05.1994 DE 4417622
(43) Veröffentlichungstag der Anmeldung: 05.03.1997
(73) Patentinhaber: BASF AKTIENGESELLSCHAFT, 67056 Ludwigshafen (DE)
(72) Erfinder: BRUDERMÜLLER, Martin, D-68239 Mannheim (DE); IRGANG, Matthias, D-69121 Heidelberg (DE); SCHMIDT-RADDE, Martin, D-67259 Beindersheim (DE); MERGER, Franz, D-67227 Frankenthal (DE); WITZEL, Tom, D-67069 Ludwigshafen (DE); KRATZ, Detlef, D-69121 Heidelberg (DE); DANZ, Eckehard, D-67071 Ludwigshafen (DE); WITTWER, Arnold, D-67434 Neustadt (DE); HESSE, Michael, D-67105 Schifferstadt (DE); SAUERWALD, Manfred, D-67149 Meckenheim (DE); FREIRE ERDBRÜGGER, Cristina, D-67240 Bobenheim-Roxheim (DE)
(74) Vertreter: Isenbruck, Günter, Dr.
(86) Internationale Anmeldenummer: EP9501920
(87) Internationale Veröffentlichungsnummer: WO9532171

(56) Entgegenhaltungen:
- EP-A- 0 020 048
- EP-A- 0 044 444
- DE-A- 3 933 661
- DE-B- 1 643 856
- DE-B- 2 538 253
- GB-A- 570 669
- US-A- 2 040 913

## Beschreibung

Vorliegende Erfindung bezieht sich auf die Herstellung von Alkoholen durch katalytische Hydrierung der entsprechenden Carbonylverbindungen. Insbesondere bezieht sich die Erfindung auf die Verwendung neuartiger Katalysatoren für diese Hydrierung.

Die vorliegende Erfindung stellt insbesondere ein neuartiges Verfahren zur Herstellung von Diolen aus den entsprechenden Aldehyden bereit. Besonderes Anwendungsgebiet ist die Herstellung von Propandiolen.

Propandiole und ihr technisch wichtigster Vertreter, das Neopentylglykol, sind Zwischenprodukte mit einem breiten Spektrum von Anwendungen in der Kunststoffindustrie, insbesondere bei Synthesefasern, Polyurethanen und Weichmachern.

Die Herstellung von Neopentylglykol erfolgt durch Kondensation von Isobutyraldehyd mit Formaldehyd und anschließende katalytische Hydrierung des entstandenen Hydroxypivalinaldehyds. Diese Hydrierung ist bereits vielfach beschrieben worden.

Als katalytisch wirksame Elemente für die Hydrierung von Alkoholen sind Ni, Ru, Pt, Re und Cu bereits bekannt.

Ni-haltige Katalysatoren sind in den Patenten DE 19 57 592 (1971), DE 20 54 601 (1972) und SE 454 171 (1988) genannt. In DE 16 43 856 wird die Hydrierung von gesättigten und ungesättigten Aldehyden zu Alkoholen an kombinierten Cu/Ni-Trägerkatalysatoren in der Gasphase beschrieben. Ein Nachteil Ni-haltiger Katalysatoren ist jedoch ihre erhebliche Neigung zur Bildung von Nebenprodukten, wie sie in EP 0 044 412 dargestellt ist.

Edelmetall-haltige Katalysatoren werden in EP 0 343 475 (Pt-Ru-W), EP 0 373 938 (Re) und SU 361998 (Ru-Ni-Cr) beschrieben. Aufgrund ihres hohen Preises sind diese Katalysatoren für den großtechnischen Einsatz allerdings wenig geeignet.

Kupfer-haltige Katalysatoren bilden aufgrund ihrer vielfältigen Vorteile gegenüber Nickel und Edelmetall enthaltenden Katalysatoren die praktisch wichtigste Gruppe von Hydrier-Katalysatoren für die Alkoholherstellung. Dabei werden vor allem Cu-Cr-Katalysatoren vielfach eingesetzt. Diese weisen allerdings den Nachteil auf, daß nur mit relativ hohen Drücken und Temperaturen während der Hydrierung hohe Raum-Zeit-Ausbeuten zu erreichen sind. Katalysatoren dieses Typs werden in US 4,855,515, DE 18 04 984 und JP 49 011 684 beschrieben.

Auch bei diesen Katalysatoren wurde aber eine erhöhte Neigung zur Bildung von Nebenprodukten beobachtet und in DE 40 37 729 und US 4,666,879 beschrieben.

Die Eigenschaften des Katalysators hängen aber nicht nur von der Wahl der katalytisch wirksamen Komponente, sondern auch entscheidend von der Wahl des Trägermaterials ab.

So werden Kupfer-Katalysatoren mit Al₂O₃ oder ZnO enthaltenden Trägermaterialien in den EP 0 044 444 (CuO/Al₂O₃), EP 0 044 412 (CuO/ZnO) und EP 0 484 800 (CuO/ZnO/ZrO₂) beschrieben. Dabei zeigen die CuO/Al₂O₃- und die CuO/ZnO-Katalysatoren zwar anfangs hohe Aktivität und Selektivität, aber auch eine starke Alterung. Ein weiteres Problem bei Al₂O₃-haltigen Katalysatoren stellt die bei höheren Temperaturen auftretende Rehydratisierung dar, die durch den Einsatz des Eduktes in wäßriger Lösung bedingt ist und zu einem Zerfall der Katalysatoren führen kann.

Durch Einsatz von ZrO₂-haltigem Trägermaterial erhält man zwar stabile Katalysatoren, die allerdings infolge des hohen Gehalts an ZrO₂ ein fast doppelt so großes Schüttgewicht wie die Al₂O₃-haltigen Katalysatoren aufweisen.

Die Eigenschaften der verwendeten Kupfer-Katalysatoren hängen aber neben der Wahl der Aktivkomponente und der Gesamtzusammensetzung auch entscheidend von den Bedingungen des Herstellungsverfahrens ab. Eine Reihe von Verfahren zur Herstellung von Kupfer-Katalysatoren für die Hydrierung von Carbonylverbindungen ist bereits bekannt.

So beschreibt DE 41 42 897 die Herstellung von Kupfer-Katalysatoren mit kleinen Kupferpartikeln und einer entsprechend hohen wirksamen Oberfläche des Katalysatormaterials durch Auffällung der kupferhaltigen Komponente. Dieses Verfahren führt jedoch trotz erheblichen Aufwandes in der Regel zu unbefriedigenden Ergebnissen.

Nach EP 0 006 313 können kleine Kupfer-Kristallite auch durch Auffällung einer Kupferverbindung bei erhöhter Temperatur auf ein inertes Trägermaterial erreicht werden.

Die Herstellung von Katalysatoren mit hohen Kupfergehalten durch Auffällung wird darüber hinaus auch in FR 1520584 beschrieben.

In US 3,701,739 wird ein Verfahren zur Herstellung von Cu/Zn-Katalysatoren durch Agglomeration von Kupfer-haltigen Teilchen im Wirbelbett und anschließende Zersetzung der Agglomerationskomponenten beschrieben. Nachteil derart hergestellter Katalysatoren ist aber ihre geringere mechanische Härte.

Wichtige Kriterien für die Eignung der in diesen Verfahren einsetzbaren Katalysatoren sind nicht nur deren Aktivität und Selektivität für die katalysierte Umsetzung, sondern auch deren mechanische Eigenschaften, wie Härte und Abriebfestigkeit. Die mechanischen Eigenschaften der verwendeten Katalysatoren sind für die Wirtschaftlichkeit des Verfahrens deshalb von besonderer Bedeutung, weil eine ungenügende mechanische Stabilität des Katalysators in relativ kurzer Zeit zur Verstopfung der Anlage infolge hohen Katalysatorabriebs führen kann, was zu langen Abschaltzeiten führt.

Der vorliegenden Erfindung lag daher die Aufgabe zugrunde, ein Verfahren zu finden, das die Herstellung von Alkoholen aus den entsprechenden Carbonylverbindungen wirtschaftlich, mit guter Ausbeute und Selektivität ermöglicht. Hierzu sollten geeignete Katalysatoren gefunden werden, die diese Umsetzung mit hoher Aktivität und Selektivität bewirken und sich durch gute mechanische Eigenschaften, insbesondere hohe Härte und Abriebfestigkeit und, vorteilhafterweise, durch ein niedriges Schüttgewicht auszeichnen sollten. Ferner sollten diese Katalysatoren preisgünstig herstellbar sein.

Diese Aufgabe wird durch das in den Ansprüchen beschriebene Verfahren zur Herstellung von Alkoholen durch katalytische Hydrierung der entsprechenden Carbonylverbindungen bei erhöhter Temperatur und bei erhöhtem Druck in flüssiger Phase gelöst. Erfindungsgemäß wird dabei ein Katalysator beschrieben, der Kupfer auf einem SiO₂-haltigen Trägermaterial in An- oder Abwesenheit eines oder mehrerer der Elemente Magnesium, Barium, Zink oder Chrom enthält, sowie das Verfahren für dessen Herstellung.

Der in dem erfindungsgemäßen Verfahren zur Herstellung von Alkoholen verwendete Katalysator zeichnet sich dadurch aus, daß die Aktivkomponente Kupfer auf einen SiO₂-haltigen Träger aufgebracht wird.

Das für einen Katalysator verwendete Trägermaterial wird als SiO₂-haltig bezeichnet, wenn es SiO₂ oder ein Silikat wie etwa Magnesiumsilikat enthält. Da die anionischen Silikatgruppen in monomerer, oligomerer und polymerer Form nebeneinander im Katalysator vorliegen, werden sie analytisch als SiO₂ erfaßt und berechnet.

Der für das erfindungsgemäße Verfahren verwendete Kupfer-Katalysator mit SiO₂-haltigem Trägermaterial enthält vorzugsweise 5 bis 75 Gew.-% Kupfer, berechnet als CuO und 95 bis 25 Gew.-% Si, berechnet als SiO₂, jeweils bezogen auf das Gesamtgewicht des calcinierten Katalysators.

Vorteilhaft im erfindungsgemäßen Verfahren einsetzbare Katalysatoren sind weiterhin solche, die außer Kupfer und Silicium eines oder mehrere der Elemente Magnesium, Barium, Zink oder Chrom enthalten. Dabei ist Magnesium in einer Menge von 0 bis 20 Gew.-%, berechnet als MgO, Barium in einer Menge von 0 bis 5 Gew.-%, berechnet als BaO, Zink in einer Menge von 0 bis 5 Gew.-%, berechnet als ZnO, und Chrom in einer Menge von 0 bis 5 Gew.-%, berechnet als Cr₂O₃, jeweils bezogen auf das Gesamtgewicht des calcinierten Katalysators, mit der Maßgabe enthalten, daß die Summe der Katalysatorbestandteile Kupfer, Silicium und, soweit enthalten, Magnesium, Barium, Zink und Chrom 100 Gew.-% ergibt.

Die beschriebenen Kupfer-Katalysatoren mit SiO₂-haltigem Trägermaterial können nach den bereits bekannten und oben erwähnten Verfahren hergestellt werden.

Insbesondere kommen folgende Herstellungsverfahren in Betracht:
- Aufbringung einer wäßrigen Kupfersalzlösung in einer oder mehreren Tränkstufen auf einen vorgefertigten Träger bestehend aus SiO₂, Magnesiumsilikat oder einem anderen schwerlöslichen Silikat. Bevorzugtes Trägermaterial ist SiO₂. Der getränkte Träger wird anschließend getrocknet und calciniert.
   Die Tränkung kann nach der sogenannten "incipient wetness"-Methode, bei der der Träger entsprechend seiner Wasseraufnahmekapazität maximal bis zur Sätttigung mit der Tränklösung befeuchtet wird. Die Tränkung kann aber auch in überstehender Lösung erfolgen.
   Bei mehrstufigen Tränkverfahren ist es zweckmäßig, zwischen den einzelnen Tränkschritten zu trocknen und gegebenenfalls zu calcinieren. Die mehrstufige Tränkung ist insbesondere dann vorteilhaft, wenn der Träger mit einer größeren Kupfermenge beaufschlagt werden soll.
   Dabei kann auch ein pulverförmiger Träger, z.B. SiO₂-Pulver oder Erdalkalisilikat-Pulver verwendet werden. Dieser wird im allgemeinen nach der Tränkung verknetet, verformt und calciniert.
- Fällung einer schwerlöslichen Kupferverbindung wie Kupfercarbonat oder Kupferhydroxid aus einer wäßrigen Lösung auf pulverförmiges SiO₂ oder Erdalkalisilikat, das als Suspension vorgelegt wird.
   Der Fällkatalysators kann auch zweistufig hergestellt werden, indem zuerst der Träger aus Wasserglas durch Säurezusatz ausgefällt und in einer zweiten Stufe ein schwerlösliches Kupfersalz aufgefällt wird.
   Die Fällung der schwerlöslichen Kupfer- und Siliciumverbindungen kann auch simultan durch Einleiten einer Kupfersalzlösung in eine Wasserglaslösung erfolgen.
   Ausgefällte Niederschläge werden in üblicher Weise filtriert, vorzugsweise alkalifrei gewaschen, getrocknet und gegebenenfalls calciniert. Die Verformung erfolgt anschließend durch Verknetung und Extrusion oder durch Tablettierung.

Besonders vorteilhaft werden im erfindungsgemäßen Verfahren Kupfer-katalysatoren verwendet, die durch Tränkung von Siliciumdioxid mit einer überstehenden wäßrigen Lösung einer thermisch leicht zersetzbaren Kupferverbindung, anschließende Trocknung und Calcinierung hergestellt worden sind. Die Calcinierung erfolgt im allgemeinen bei Temperaturen von 200 bis 400°C, vorzugsweise von 250 bis 350°C.

Solche Kupferkatalysatoren enthalten im allgemeinen 5 bis 50, vorzugsweise 5 bis 30 Gew.-% Kupfer, berechnet als CuO, und 50 bis 95, vorzugsweise 70 bis 95 Gew.-% Silicium, berechnet als SiO₂, jeweils bezogen auf das Gesamtgewicht des calcinierten Katalysators.

Die Tränkung mit einer überstehenden Lösung führt zu einer relativ gleichmäßigen Durchtränkung des Trägermaterials und damit zu einer feineren und gleichmäßigeren Verteilung des Kupfers im Träger. Die Ausbildung von ungleichförmigen Konzentrationsprofilen über den Querschnitt des Trägermaterials wie sie bei der Tränkung nach der sogenannten "incipient wetness"-Methode auftreten können, werden bei diesem Verfahren vermieden, wodurch die Herstellung der erfindungsgemäß zu verwendenden Katalysatoren reproduzierbarer und folglich wirtschaftlicher wird.

Die Verwendung von Kupferverbindungen, die bei Temperaturen von 200 bis 400, vorzugsweise von 250 bis 350 °C zersetzbar sind, erlaubt entsprechend geringere Calcinierungstemperaturen. Dadurch wird die Bildung kleinerer Kupferteilchen und deren gleichmäßige Verteilung auf dem Trägermaterial begünstigt.

Die nach dem neuartigen Verfahren hergestellten Katalysatoren weisen deshalb eine vergleichsweise höhere wirksame Kupferoberfläche auf. Dabei bieten sowohl die Tränkung in überstehender Lösung eines herkömmlicherweise verwendeten Kupfersalzes, wie Kupfernitrat, wie auch die Verwendung von bei geringerer Temperatur zersetzlichen Kupferverbindungen für sich jeweils die angegebenen Vorteile. Der beste Effekt wird aber erzielt, wenn in überstehender Lösung getränkt wird und dabei eine leicht zersetzliche Kupferverbindung eingesetzt wird.

Ein Maß für die wirksame Kupferoberfläche bei einer bestimmten Menge an eingesetztem Kupfer ist die Dispersion. Sie ist definiert als Verhältnis der Zahl der Kupfer-Atome an der Oberfläche der Kupfer-Kristallite zur Gesamtzahl der Kupfer-Atome in den Kupfer-Kristalliten des Trägers.

Die Dispersion des Kupfers in einem Katalysator kann direkt aus der Größe der Kristallite oder indirekt aus der zur Oxidation der Kupferoberfläche nötigen Menge an Sauerstoff und der bei der Tränkung verwendeten Menge an Kupfer bestimmt werden.

Die direkte Ermittlung der Größe der Kupfer-Kristallite kann durch Transmissionselektronenmikroskopie (TEM) an einer im Ultramikrotom nach Fixierung mit Polymethylmethacrylat hergestellten ultradünnen Scheibe des fertigen Kupfer-Katalysators erfolgen. Partikelgrößen bis zu 1 nm können so bestimmt werden. Aus der Größe der Kupfer-Kristallite kann dann unmittelbar die Dispersion des Kupfers errechnet werden.

Ergänzend kann die Phase der kupferhaltigen Partikel durch Energy Dispersive X-ray Spectroscopy (EDX-S) oder Selected Area Diffraction (SAD) bestimmt werden.

Bei dem indirekten Verfahren zur Ermittlung der Dispersion errechnet man aus der Menge des bei der Oxidation der Kupferoberfläche verbrauchten Sauerstoffs und der Menge des im Träger enthaltenen Kupfers dessen Dispersion im Katalysator-Träger.

Dabei wird eine genau abgewogene Probe des fertigen Katalysators nach einstündiger Spülung mit Helium auf 200°C im Vakuum erhitzt und anschließend 16 Stunden lang mit Wasserstoff reduziert. Nach vollständiger Reduktion wird nochmals evakuiert. Dann läßt man die Probe auf 35°C erkalten und ermittelt die Sauerstoffchemisorption durch ein volumetrisches Sorptionsverfahren, wobei die Sauerstoffchemisorption an mindestens vier verschiedenen Punkten im Druckbereich von 50 bis 250 Torr (66,65 bis 33,25 mbar) gemessen wird (Gerät: z.B. Chemisorb 2810 der Fa. Micromeretics). Ausgehend von der Annahme, daß je zwei Kupfer-Atome mit einem Sauerstoff-Atom reagieren, kann die Kupferoberfläche berechnet werden. Die Kupfermenge in der Katalysatorprobe kann durch einfache Analytik bestimmt werden. Aus dem Verhältnis der so ermittelten Anzahl der an der Kupferüberfläche liegenden Kupferatome zur Gesamtzahl der Kupferatome in der vermessenen Katalysatorprobe multipliziert mit 100 ergibt sich die Dispersion des Kupfers im Katalysator in %.

Neben einer erhöhten Dispersion weisen die nach dem neuartigen Verfahren hergestellten Katalysatoren aber auch eine verbesserte mechanische Stabilität auf. Kennzeichnend für die erhöhte mechanische Stabilität sind vor allem eine größere Härte und ein verminderter Abrieb des Katalysators.

Diese Parameter können wie folgt bestimmt werden. Zur Ermittlung der Schneidhärte werden Proben mit einer Schneide zertrennt. Die Kraft mit der die Schneide belastet werden muß, um eine Durchtrennung der Probe zu Erreichen bezeichnet man als Schneidhärte des Materials.

Die Bruchhärte von kugelförmigen Proben wird dadurch ermittelt, daß man die Kugel unter einen Stempel mit definierter Fläche legt und den Stempel dann solange gegen die Kugel bewegt bis sie zerbricht. Der mit dem Stempel auf die Probe ausgeübte Druck, der zur Erzielung des Bruches notwendig ist, wird als Bruchhärte bezeichnet.

Der Abrieb wird einer Schwingmühle bestimmt. Dabei wird Katalysatormaterial eines bestimmten Korngrößenbereichs zusammen mit Porzellankugeln in einem Behälter bei hoher Drehzahl für eine bestimmte Zeitdauer bewegt. Danach wird der Katalysator wieder abgesiebt. Der Gewichtsverlust in Prozent wird dann als Abrieb bezeichnet.

Vorzugsweise wird im Rahmen des erfindungsgemäßen Verfahrens ein Katalysator verwendet, der durch Tränkung eines porösen Siliciumdioxid-Trägermaterials mit einer überstehenden wäßrigen Lösung einer thermisch leicht zersetzbaren Kupferverbindung unter Anwendung einer Tränkzeit von 2 bis 60, vorzugsweise von 5 bis 30 Minuten, anschließende Trocknung und Calcinierung, erhalten worden ist.

Insbesondere werden bevorzugt solche Katalysatoren im erfindungsgemäßen Verfahren verwendet, die durch Tränkung eines porösen Siliciumdioxid-Trägermaterials mit einer BET-Oberfläche von mehr als 100 m²/g mit einer überstehenden wäßrigen Lösung einer thermisch leicht zersetzbaren Kupferverbindung, anschließende Trocknung und Calcinierung erhalten worden sind.

Unter thermisch leicht zersetzbaren Kupferverbindungen werden Kupferverbindungen verstanden, die sich bei Calciniertemperaturen von 200 bis 400°C, vorzugsweise von 250 bis 350°C, zu oxidischen Kupferverbindungen oder elementarem Kupfer zersetzen. Beispielhaft für solche Verbindungen seien Kupfercarbonat, Kupferoxalat oder Kupferformiat genannt. Da diese Kupfersalze als solche teilweise schwer löslich sind und die Lösungen mancher dieser Salze zur Hydrolyse neigen, werden diese Kupfersalze vorzugsweise in Form ihrer relativ stabilen, gut wasserlöslichen Komplexe mit Ammoniak, Hydrazin oder Aminen, vorzugsweise in Form ihrer Ammin-Komplexe, in der Tränklösung eingesetzt. Besonders bevorzugt wird hierzu ammoniakalische Kupfercarbonat-Lösung verwendet. Die Verwendung dieser thermisch leicht zersetzbaren Kupferverbindungen ermöglicht die Calcinierung bei den genannten niedrigen Temperaturen, während herkömmlicherweise zur Tränkung eingesetzte Kupfersalze, wie Kupfernitrat oder Kupfersulfat, im allgemeinen Calciniertemperaturen um 500°C erfordern. Sowohl die Verwendung thermisch leicht zersetzbarer Kupferverbindungen als auch die Anwendung niedriger Calciniertemperaturen ermöglicht die Erzeugung kleiner Kupferkristallite und damit einer höheren katalytisch wirksamen Kupferoberfläche im fertigen Katalysator.

Im Rahmen vorliegender Erfindung können deshalb grundsätzlich alle bei geringeren Temperaturen, d.h. bei weniger als 400 °C bei Normaldruck zersetzlichen Kupferverbindungen verwendet werden.

Das erfindungsgemäße Verfahren kann unter Verfahrensbedingungen, wie sie üblicherweise in Hydrierverfahren angewandt werden, durchgeführt werden. Die im folgenden genannten Bedingungen sind jedoch bevorzugt.

Der Hydrierungsreaktor kann in Sumpf- oder Rieselfahrweise betrieben werden. Dabei ist es in der Regel vorteilhaft einen Teil des Hydrierungsproduktes in den Hydrierprozeß zurückzuführen (Kreislauffahrweise).

Vorzugsweise wird der Katalysator vor seiner Verwendung im erfindungsgemäßen Verfahren mit reduzierenden Gasen, beispielsweise Wasserstoff, vorzugsweise mit Wasserstoff-Inertgasgemischen, insbesondere Wasserstoff-Stickstoff-Gemischen, bei Temperaturen von 100 bis 300, vorzugsweise von 150 bis 250 °C, vorreduziert.

Die Katalysatorbelastung beträgt bei der Hydrierung vorzugsweise 0,1 bis 1,0 1_{Carbonylverbindung}/1_{Kat.}*h. Pro Stunde fließen somit 0,1 bis 1 Liter Carbonylverbindung durch ein Katalysatorvolumen von 1 Liter.

Vorzugsweise werden die Carbonylverbindungen bei einer Temperatur von 60 bis 200, vorzugsweise von 80 bis 160 °C und bei einem Druck von 1 bis 150, vorzugsweise von 20 bis 100 bar hydriert, wobei in der flüssigen Phase ein pH-Wert von 4 bis 13, vorzugsweise von 6 bis 12, besonders bevorzugt von 7 bis 12, eingehalten wird.

Das erfindungsgemäße Verfahren kann grundsätzlich für die Hydrierung beliebiger Carbonylverbindungen zu Alkoholen verwendet werden. Für die Hydrierung einiger im folgenden genannten Carbonylverbindungen ist das erfindungsgemäße Verfahren aber besonders vorteilhaft.

Beispielsweise können nach dem erfindungsgemäßen Verfahren vorteilhaft C₂- bis C₂₀-Hydroxyaldehyde bzw. -ketone zu den entsprechenden Alkoholen hydriert werden. Dabei können diese Hydroxycarbonylverbindungen eine oder mehrere Hydroxylgruppen enthalten. So ist das erfindungsgemäße Verfahren sehr gut für die Hydrierung von Polyhydroxycarbonylverbindungen, insbesondere von Kohlenhydraten, wie Glucose, Fructose, Mannose oder Xylose geeignet, wobei aus diesen Zuckern die entsprechenden Zuckeralkohole, wie Sorbit, Mannit oder Xylit gebildet werden, die als Zuckeraustauschstoffe Verwendung finden.

Besonders vorteilhaft wird das erfindungsgemäße Verfahren zur Hydrierung von C₂- bis C₂₀-Monohydroxyaldehyden zu den entsprechenden Alkoholen eingesetzt. Dabei werden vorzugsweise Hydroxypropionaldehyde der allgemeinen Formel I 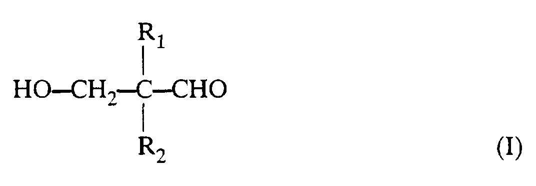 als Carbonylverbindungen eingesetzt und daraus 1,3-Propandiole der allgemeinen Formel II 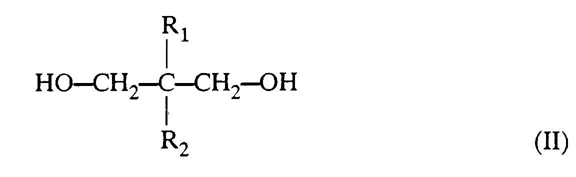 hergestellt, worin die Reste R¹ und R² gleich oder verschieden sind und jeweils für Wasserstoff, eine C₁- bis C₂₄-Alkyl-, eine C₆- bis C₂₀-Aryl-und/oder eine C₇- bis C₁₂-Aralkylgruppe stehen oder beide Reste R¹ und R² gemeinsam mit den benachbarten Kohlenstoffatomen einen 5 bis 10-gliedrigen alicyclischen Ring bilden.

Besonders vorteilhaft wird im erfindungsgemäßen Verfahren als Carbonylverbindung Hydroxypivalinaldehyd (HPA) der Formel Ia 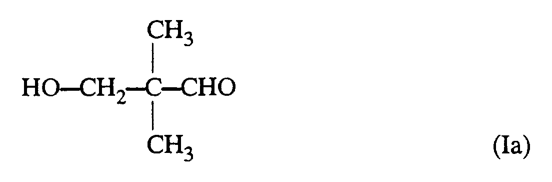 eingesetzt und daraus Neopentylglykol (NPG) der Formel IIa 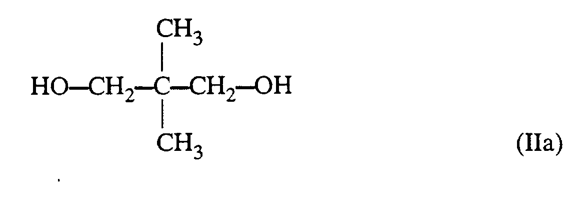 hergestellt. Die Hydrierausbeute ist dabei außerordentlich hoch, bei Verwendung der bevorzugten Katalysatoren sogar nahe 100%, auch bei sehr hohen Belastungen. Eine die Wirtschaftlichkeit des Verfahrens beeinträchtigende Bildung von Nebenprodukten wie Estern und Acetalen konnte dabei nicht festgestellt werden. Ebensowenig konnte eine Rückspaltung von Hydroxypivalinaldehyd in Isobutyraldehyd und Formaldehyd beobachtet werden.

In einer bevorzugten Ausführungsform des Verfahrens zur Herstellung von NPG wird nacheinander
- Isobutyraldehyd und Formaldehyd in wäßrigem Medium in Gegenwart eines Trialkylamins zu HPA umgesetzt,
- nicht umgesetztes Isobutyraldehyd und, soweit technisch unvermeidlich, Trialkylamin aus der wäßrigen Lösung, vorzugsweise durch Destillation, von der Reaktionslösung abgetrennt,
- vorzugsweise zu der von nicht umgesetztem Isobutyraldehyd befreiten HPA-Lösung frisches oder abgetrenntes Trialkylamin zugesetzt bis in der Lösung ein pH-Wert von 4 bis 13, vorzugsweise von 6 bis 12, besonders bevorzugt von 7 bis 12, erreicht ist,
- die so erhaltene HPA-Lösung, die neben Hydroxypivalinaldehyd, Trialkylamin und andere Verunreinigungen und Nebenprodukte aus der Umsetzung von Isobutyraldehyd mit Formaldehyd enthält, mit Wasserstoff in Gegenwart eines erfindungsgemäß einzusetzenden Kupfer-Katalysators hydriert, und
- aus der hydrierten Lösung Neopentylglykol, vorzugsweise durch Destillation, gewonnen.

Das bei der Destillation der Reaktionslösung aus der Kondensation von Isobutyraldehyd mit Formaldehyd erhaltene Isobutyraldehyd/Trialkylamin-Gemisch kann vorteilhaft erneut für die Kondensationsreaktion eingesetzt werden. Die Kondensationsreaktion von Isobutyraldehyd mit Formaldehyd kann vorzugsweise nach dem Verfahren ausgeführt werden, wie es in US-A 3,808,280 beschrieben ist, worauf hiermit Bezug genommen wird.

Weiter bevorzugt wird als Carbonylverbindung 2-Hydroxymethyl-2-methylbutanal der Formel Ib 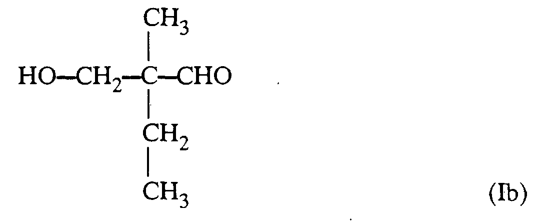 eingesetzt und hieraus 2-Methyl-2-ethyl-propandiol-1,3 der Formel IIb 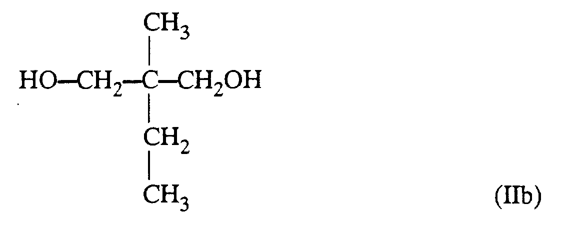 erzeugt.

Besonders bevorzugt ist demgegenüber die Verwendung von 1-Formyl-1-hydroxymethylcyclopentan der Formel Ic als Carbonylverbindung.

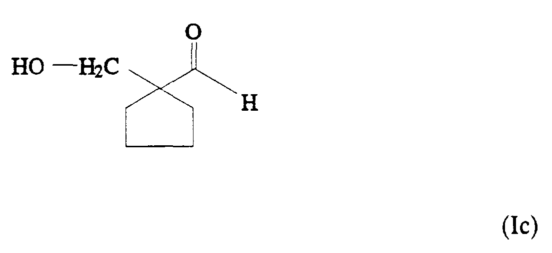

Daraus gewinnt man 1,1-Bis(hydroxymethyl)cyclopentan der Formel IIc.

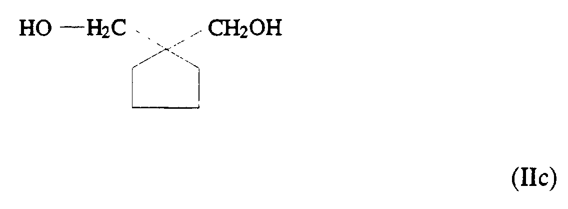

Zu betonen ist, daß die obigen Darlegungen lediglich beispielhaft der Erläuterung der praktisch universellen Anwendbarkeit des erfindungsgemäßen Verfahrens zur Herstellung von Alkoholen aus Hydroxycarbonylverbindungen dienen.

Vorteilhaft können mit Hilfe des erfindungsgemäßen Verfahrens auch die entsprechenden Alkohole aus anderen Carbonylverbindungen, z.B. geradkettigen oder verzweigten aliphatischen Aldehyden mit 1 bis 24 Kohlenstoffatomen, geradkettigen oder verzweigten aliphatischen Ketonen mit 1 bis 24 Kohlenstoffatomen oder alicyclischen C₄- bis C₁₂-Ketonen, hergestellt werden. Die hierbei gewonnenen Alkohole dienen zum Beispiel als Lösungsmittel oder als Zwischenprodukte bei der Herstellung von Tensiden und Dispersionen. Lediglich beispielhaft sei die Hydrierung von Propionaldehyd zu n-Propanol, Butyraldehyd zu n-Butanol, Isobutyraldehyd zu Isobutanol, Valeraldehyd zu n-Pentanol, 2-Ethylhexanal zu 2-Ethylhexanol, Aceton zu Isopropanol, Methylethylketon zu 2-Butanol, Cyclopentanon zu Cycolpentanol und von Cyclohexanon zu Cyclohexanol genannt. Es können auch Gemische dieser Carbonylverbindungen als Ausgangsmaterial im erfindungsgemäßen Verfahren eingesetzt werden. Ein besonderer Vorteil des erfindungsgemäßen Verfahrens besteht darin, daß die erfindungsgemäß zu verwendenden Kupferkatalysatoren relativ unempfindlich gegenüber typischen Verunreinigungen der Hydrierungsedukte, z.B.

Acetale, Carbonsäuren, etc., sind. Die Ausgangsstoffe können somit ohne aufwendige Vorreinigung im erfindungsgemäßen Verfahren eingesetzt werden. So können z.B. Butanal/Isobutanal-Gemische, wie sie bei der Hydroformylierung von Propen erhalten werden, direkt der Hydrierung zugeführt werden.

Das erfindungsgemäße Verfahren eignet sich auch sehr gut für die Herstellung der entsprechenden gesättigten Alkohole aus geradkettigen oder verzweigten aliphatischen C₃- bis C₁₂-α,β-ungesättigten Carbonylverbindungen. Beispielhaft sei hier die Hydrierung von Acrolein zu n-Propanol, von Crotonaldehyd zu n-Butanol, von 2-Ethylhex-2-en-1-al zu 2-Ethylhexanol und von 2-Propylhept-2-en-1-al zu 2-Propylheptanol genannt. 2-Ethylhexanol und 2-Propylheptanol sind technisch wichtige Weichmacheralkohole. Auch bei der Hydrierung dieser α,β-ungesättigten Carbonylverbindungen können die Ausgangsstoffe in technischer Qualität, ohne vorausgehende Feinreinigung, dem erfindungsgemäßen Verfahren zugeführt werden, ohne daß sich dies nachteilig auf das Verfahrensergebnis auswirkt.

### Beispiel 1

Tabelle I zeigt einen Vergleich der für das erfindungsgemäße Verfahren vorgesehenen Kupfer-Katalysatoren auf einem SiO₂-haltigen Träger mit herkömmlichen Kupfer-Katalysatoren am Beispiel der Hydrierung von Hydroxypivalinaldehyd.

Dabei sind die Katalysatoren in der folgenden Weise hergestellt:

### Katalysator A

1000 g eines handelsüblichen SiO₂-Katalysator-Trägers mit einer Porosität von 1,0 ml/g und einer BET-Oberfläche von etwa 200 m²/g wurden mit 1000 ml einer ammoniakalischen Kupfercarbonat-Lösung getränkt. Nach vollständiger Aufnahme der Lösung wurde der Träger getrocknet und bei 500 °C calciniert.
Dann wurde in gleicher Weise eine zweite Tränkung vorgenommen und wiederum getrocknet und calciniert.
Der fertige Katalysator enthielt 25 Gew.-% CuO, bezogen auf das Gesamtgewicht des Katalysators. Er wies ein Schüttgewicht von 625 g/l, eine BET-Oberfläche von 150 m²/g und eine Porosität von 0,6 ml/g auf. Katalysator A hatte eine Schneidhärte von 18 N.

### Katalysator B

1,5 kg einer pulverförmigen Fällungskieselsäure (BASF, D11-10 Pulver) wurden in einem Kneter langsam mit 2,8 kg einer ammoniakalischen Kupfercarbonat-Lösung versetzt, die 14,3 Gew.-% Cu, bezogen auf das Gesamtgewicht der Lösung, enthielt. Nach einstündigem Kneten wurde die Masse mit einem Schneckenextruder verformt, anschließend getrocknet und bei 500 °C calciniert.
Der Katalysator hatte ein Schüttgewicht von 475 g/l und enthielt 24 Gew.-% CuO, bezogen auf das Gesamtgewicht des Katalysators. Katalysator B hatte eine Schneidhärte von 28,4 N.

### Katalysator C

In einem Rührgefäß wurde bei Raumtemperatur gleichzeitig eine Lösung von Natronwasserglas, die 1,9 kg SiO₂ enthielt, und eine Lösung von Magnesiumnitrat mit umgerechnet 1,0 kg MgO eingepumpt. Dabei stellte sich ein pH-Wert von etwa 10 ein. Anschließend wurde gleichzeitig eine Mischlösung von Kupfernitrat/Bariumnitrat/Zinknitrat/Chromnitrat sowie eine Soda-Lösung zugegeben, sodaß ein pH-Wert von 7,8 eingestellt wurde.
Der gesamte Niederschlag wurde auf einer Filterpresse ausgewaschen, der Filterkuchen getrocknet, bis zu einem Glühverlust von 15% calciniert und das so entstandene Pulver tablettiert.
Der fertige Katalysator enthielt 60 Gew.-% CuO, 13 Gew.-% MgO, 25 Gew.-% SiO₂, 1 Gew.-% BaO, 0,5 Gew.-% ZnO, 0,5 Gew.-% Cr₂O₃, jeweils bezogen auf das Gesamtgewicht des Katalysators. Er hatte ein Schüttgewicht von 1050 g/l. Die Schneidhärte betrug 97 N, der Abrieb 6%.

### Katalysator D = Vergleichsbeispiel

Eine Kupfernitratlösung wurde mit einer Ammoniumbicarbonat-Lösung versetzt und die erhaltene Suspension mit einer ZnO-Suspension vermischt und 40 Minuten lang bei 70 °C mit Kohlendioxid begast. Zu dieser Mischung wurde eine Zirkonylnitrat-Lösung und nochmals Ammoniumbicarbonat zugefügt. Nach 30 Minuten Rühren bei 40 °C wurde filtriert und gewaschen.

Das Produkt enthielt, bezogen auf das Gesamtgewicht, 31 Gew.-% CuO, 67 Gew.-% ZrO₂ und 2 Gew.-% ZnO. Es wurde getrocknet, bei 350 °C calciniert und tablettiert. Das Schüttgewicht betrug 1900 g/l.

### Katalysator E = Vergleichsbeispiel

Man stellte eine Mischlösung aus Kupfernitrat und Aluminiumnitrat her (Gew.-Verh. CuO/Al₂O₃=54/46) und pumpte diese Lösung gleichzeitig mit einer Soda-Lösung in einen Rührbehälter, sodaß ein konstanter pH-Wert von 5,5 über die Dauer der Fällung erhalten blieb. Anschließend wurde der pH-Wert auf 7,0 erhöht und 60 Minuten unter Lufteinleitung nachgerührt. Dabei stieg der pH-Wert auf 7,9. Die Temperatur im Fällbehälter wurde während der Gesamtzeit auf 70 °C gehalten. Das Fällungsprodukt wurde auf einer Filterpresse ausgewaschen, getrocknet und tablettiert. Die Tabletten wurden bei 620 °C eine Stunde lang calciniert. Der Katalysator besaß ein Schüttgewicht von etwa 1000 g/l, eine BET-Oberfläche von 80 m²/g und enthielt etwa 54 Gew.-% CuO, bezogen auf das Gesamtgewicht des Katalysators.

Zur Bestimmung der mechanischen Eigenschaften der Katalysatoren, wie sie in den folgenden Tabellen angegeben sind, wurden die oben bereits beschriebenen Verfahren benutzt. Im Einzelnen wurden die Werte wie folgt bestimmt:

Zur Bestimmung der Schneidhärte wurde ein handelsübliches Prüfgerät (Hersteller: Fa. NENE) mit einer Schneide von 0,3 mm Stärke, mit feststehender Platte und frei beweglichem horizontalem Stempel benutzt.

Die Bruchhärte wurde mit einem handelsüblichen Prüfgerät (Hersteller: Fa. Frank, Typ 1,557) mit einem Stempeldurchmesser von 3 mm gemessen.

Bei der Ermittlung des Abriebs wurde die Katalysatorprobe über ein 2 mm Sieb abgesiebt. Dann wurden 100g der Probe zusammen mit 30g Porzellankugeln mit einem Durchmesser von 10 bis 11 mm und einem Gewicht von jeweils 1,7 bis 2,0 g in einen Behälter gegeben, der auf einer Schwingmühle 2 Stunden lang mit 1400 Umdrehungen rotiert wurde. Danach wurde wieder über ein 2 mm Sieb abgesiebt.

Die Hydrierung von Hydroxypivalinaldehyd wurde unter folgenden Bedingungen vorgenommen:

Eine Menge von 60 ml der in Form von Extrudaten, Tabletten oder Kugeln vorliegenden obigen Katalysatoren wurde in einen elektrisch beheizbaren Rohrreaktor (Volumen = 100 ml) eingebaut und drucklos mit einem H₂/N₂-Gasgemisch bei Temperaturen von 150 bis 240 °C reduziert. Nach 5 Stunden wurde der Wasserstoffanteil auf 100% erhöht und anschließend ein Wasserstoffdruck von 30 bar eingestellt.

Nach der Reduktion wurde eine wäßrige Lösung mit einem Gehalt von 75 Gew.-% HPA, bezogen auf das Gesamtgewicht der Lösung, im geraden Durchgang über den Katalysator gepumpt. Das Produkt der Hydrierung wurde in einer Vorlage aufgefangen und mit Hilfe der Gaschromatographie analysiert (GC-Analyse).

Die Belastung und die eingestellten Hydriertemperaturen sind in Tabelle I zusammen mit den erzielten Umsätzen, Selektivitätswerten und der als Nebenprodukt gebildeten Isobutanol-Menge für die genannten Katalysatoren angegeben.

Die erfindungsgemäßen Katalysatoren A, B und C weisen gegenüber den herkömmlichen Katalysatoren D und E hohe Aktivität und Temperaturstabilität auf. Die Katalysatoren A und B zeigen außerdem ein deutlich niedrigeres Schüttgewicht. Katalysator A weist überdies eine hohe Selektivität und vollständigen Umsatz bis zu hohen Belastungen auf.

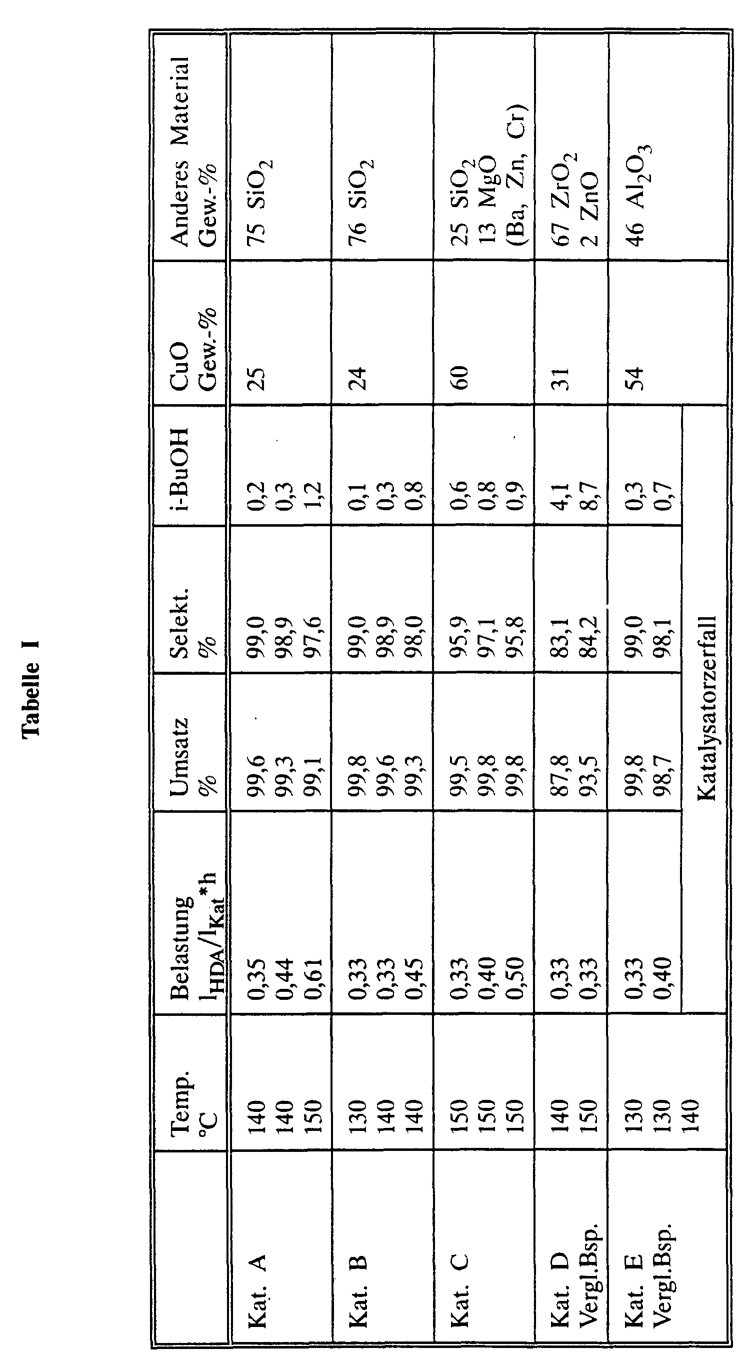

### Beispiel 2

Im Folgenden werden anhand der Hydrierung einer Reihe von Carbonylverbindungen die Eigenschaften erfindungsgemäßer und nicht erfindungsgemäßer Katalysatoren im erfindungsgemäßen Verfahren verglichen.

Die Katalysatoren werden dabei in folgender Weise hergestellt:

### Katalysator F = Vergleichsbeispiel

Die Herstellung von Katalysator F erfolgte wie die des Katalysators E in Beispiel 1.

### Katalysator G

SiO₂-Kugeln von 3 bis 5 mm Durchmesser wurden mit einer Lösung von Kupfercarbonat in konzentriertem wäßrigen Ammoniak getränkt. Diese Tränkung erfolgte 15 Minuten lang in überstehender Lösung. Die getränkten Kugeln wurden anschließend 5 Stunden lang bei 120 °C getrocknet und danach 2 Stunden lang bei 300 °C calciniert.
Diese Tränk- und Calcinierschritte wurden einmal wiederholt.
Der Kupfergehalt der Tränklösung wurde so eingestellt, daß nach einmaliger oder mehrmaliger Tränkung der gewünschte Kupfergehalt des Katalysators erhalten wurde.
Der fertige Katalysator enthielt 25,6 Gew.-% CuO und 74,4 Gew.-% SiO₂, bezogen jeweils auf das Gesamtgewicht des Katalysators.

### Katalysator H

Katalysator H wurde durch Tränkung von 3 mm dicken SiO₂-Strängen mit einer ammoniakalischen Kupfercarbonat-Lösung nach dem "incipient wetness"-Verfahren hergestellt. Die Tränklösung wurde dem Trägermaterial dabei in einer Menge zudosiert, die dessen Wasseraufnahmevermögen entsprach. Anschließend wurde getrocknet und bei 350°C calciniert.

Der fertige Katalysator enthielt 23,6 Gew.-% CuO und 76,4 Gew.-% SiO₂, jeweils bezogen auf das Gesamtgewicht des Katalysators.

### Katalysator I

Die Herstellung erfolgte analog der Herstellung von Katalysator H. Als Trägermaterial wurden SiO₂-Kugeln mit einem Durchmesser von 1,5 bis 3 mm verwendet.
Der fertige Katalysator enthielt 24,5 Gew.-% CuO und 75,5 Gew.-% SiO₂, jeweils bezogen auf das Gesamtgewicht des Katalysators.

### Katalysator J

Die Herstellung erfolgte analog der Herstellung von Katalysator H, wobei als Trägermaterial die bei Katalysator G beschriebenen SiO₂-Kugeln verwendet wurden.
Der fertige Katalysator enthielt 23,7 Gew.-% CuO und 76,3 Gew.-% SiO₂, jeweils bezogen auf das Gesamtgewicht des Katalysators.

### Katalysator K

SiO₂-Stränge mit 4 mm Durchmesser wurden mit Kupfernitratlösung getränkt, wobei die Lösung dem Träger in einer Menge zugegeben wurde, die der Wasseraufnahme des Trägermaterials entsprach (incipient wetness Verfahren). Die getränkten Stränge wurden 5 Stunden lang bei 120 °C getrocknet und danach 2 Stunden lang bei 520 °C calciniert.
Diese Tränk- und Calcinierschritte wurden einmal wiederholt.
Der fertige Katalysator enthielt 24,9 Gew.-% CuO und 75,1 Gew.-% SiO₂, jeweils bezogen auf das Gesamtgewicht des Katalysators.

### Katalysator L

SiO₂-Kugeln mit einem Durchmesser von 3 bis 5 mm wurden mit einer ammoniakalischen Kupferacetat-Lösung in überstehender Lösung für 15 Minuten getränkt. Die getränkten Kugeln wurden anschließend 5 Stunden lang bei 120 °C getrocknet und danach zwei Stunden lang bei 350 °C calciniert.
Diese Tränk- und Calcinierschritte wurden einmal wiederholt.
Der fertige Katalysator enthielt 26,3 Gew.-% CuO und 73,7 Gew-% SiO₂, jeweils bezogen auf das Gesamtgewicht des Katalysators.

### Katalysator M

Zur Herstellung wurde das gleiche Trägermaterial wie für Katalysator G verwendet. Die Tränkung erfolgte nach dem "incipient wetness"-Verfahren, d.h. die zugegebene Menge der Tränklösung entsprach der Wasseraufnahmekapazität des Trägers. Es wurde, wie im Folgenden dargestellt, mehrfach getränkt.
Im ersten Tränkschritt wurde eine Lösung von Calcium- und Chromnitrat aufgebracht, dann wurde 6 Stunden lang bei 120 °C getrocknet und 2 Stunden lang bei 700 °C calciniert.
Im zweiten Tränkschritt wurde die bei Katalysator G beschriebene Kupferlösung verwendet. Danach wurde wiederum 6 Stunden lang bei 120 °C getrocknet und 3 Stunden lang bei 350 °C calciniert.
Der fertige Katalysator enthielt 12,6 Gew.-% CuO, 5,9 Gew.-% CaO, 2,7 Gew.-% Cr₂O₃ und 78,8 Gew.-% SiO₂, jeweils bezogen auf das Gesamtgewicht des Katalysators.

### Katalysator N

SiO₂-Stränge mit 4 mm Durchmesser wurden mit einer Lösung von Kupfer-, Nickel- und Mangannitrat getränkt. Die Tränkung erfolgte durch Zugabe der Tränklösung zum Trägermaterial in einer Menge, die der Wasseraufnahme des Trägermaterials entsprach (incipient wetness Verfahren). Die Stränge wurden anschließend 5 Stunden lang bei 120 °C getrocknet und danach 2 Stunden lang bei 520 °C calciniert.
Diese Tränk- und Calcinierschritte wurden einmal wiederholt.
Der fertige Katalysator enthielt 21,3 Gew.-% NiO, 7,6 Gew.-% CuO, 2,1 Gew.-% Mn₂O₃ und 69,0 Gew.-% SiO₂, jeweils bezogen auf das Gesamtgewicht des Katalysators.

### Katalysator O

SiO₂-Stränge von 4 mm Durchmesser wurden mit einer Lösung von Kupfer-, Nickel- und Mangannitrat sowie Phosphorsäure getränkt. Die Tränkung erfolgte durch Zugabe der Tränklösung zum Trägermaterial in einer Menge, die der Wasseraufnahme dieses Materials entsprach (incipient wetness Verfahren). Die Stränge wurden danach 5 Stunden lang bei 120 °C getrocknet und 2 Stunden lang bei 520 °C calciniert.
Diese Tränk- und Calcinierschritte wurden einmal wiederholt.
Der fertige Katalysator enthielt 21,5 Gew.-% NiO, 7,5 Gew.-% CuO, 2,0 Gew.-% Mn₂O₃, 1,2 Gew.-% H₃PO₄ und 67,8 Gew.-% SiO₂, jeweils bezogen auf das Gesamtgewicht des Katalysators.

Die mechanischen Eigenschaften wurden wie bei Beispiel 1 beschrieben, bestimmt.

Die Hydrierung der bevorzugten Carbonylverbindungen erfolgte unter den folgenden Bedingungen:
Das Hydrieredukt wurde zusammen mit Wasserstoff bei einem Druck von 30 bis 50 bar in einem beheizbaren Rohrreaktor, der mit der jeweiligen Katalysatorpräparation gefüllt war, gepumpt.
Dabei wurden Reaktoren von 75 ml, 200 ml und 1000 ml Inhalt verwendet.
Das Reaktionsprodukt wurde nach der Hydrierung entspannt und in einer Vorlage aufgefangen. Die Analysen wurden mit dem Verfahren der Gaschromatographie durchgeführt.
Die zu hydrierende Lösung wurde dabei im geraden Durchgang in Sumpf- oder Rieselfahrweise über die Katalysatorschüttung gepumpt. Wahlweise wurde dabei ein Teil des Reaktionsaustrages vor der Entspannung in den Reaktor zurückgepumpt (Kreislauffahrweise).
Die Belastungen wurden auf die Volumina der zu hydrierenden Carbonylverbindung in der Lösung und der Katalysatorschüttung bezogen.

Der jeweilige Katalysator wurde entweder in reduziert-passivierter Form verwendet und direkt mit der Hydrierlösung unter Wasserstoffdruck beschickt. Oder er wurde in seiner oxidischen Form eingesetzt und vor der Zudosierung der zu hydrierenden Lösung vorreduziert. Dabei wurde die Vorreduktion mit einem Wasserstoff/Stickstoffgemisch im Verhältnis 1 : 100 bis 1 : 10 bei 180 bis 240 °C für 12 Stunden durchgeführt.

### Beispiel 2.1: Hydrierung von Hydroxypivalinaldehyd (HPA) zu Neopentylglykol (NPG)

Als Ausgangslösung diente wäßrige HPA-Lösung mit einem Wassergehalt von 24 Gew.-%, bezogen auf das Gesamtgewicht der Lösung.

Die Hydrierung erfolgte in einem Reaktorvolumen von 200 ml bei einem Katalysatorvolumen von 200 ml in der Rieselfahrweise mit Produktrückführung (Kreislauf = 9,5 l/h). Die Ergebnisse sind in Tabelle II dargestellt. Die angegebenen Katalysatoren beziehen sich dabei auf HPA.

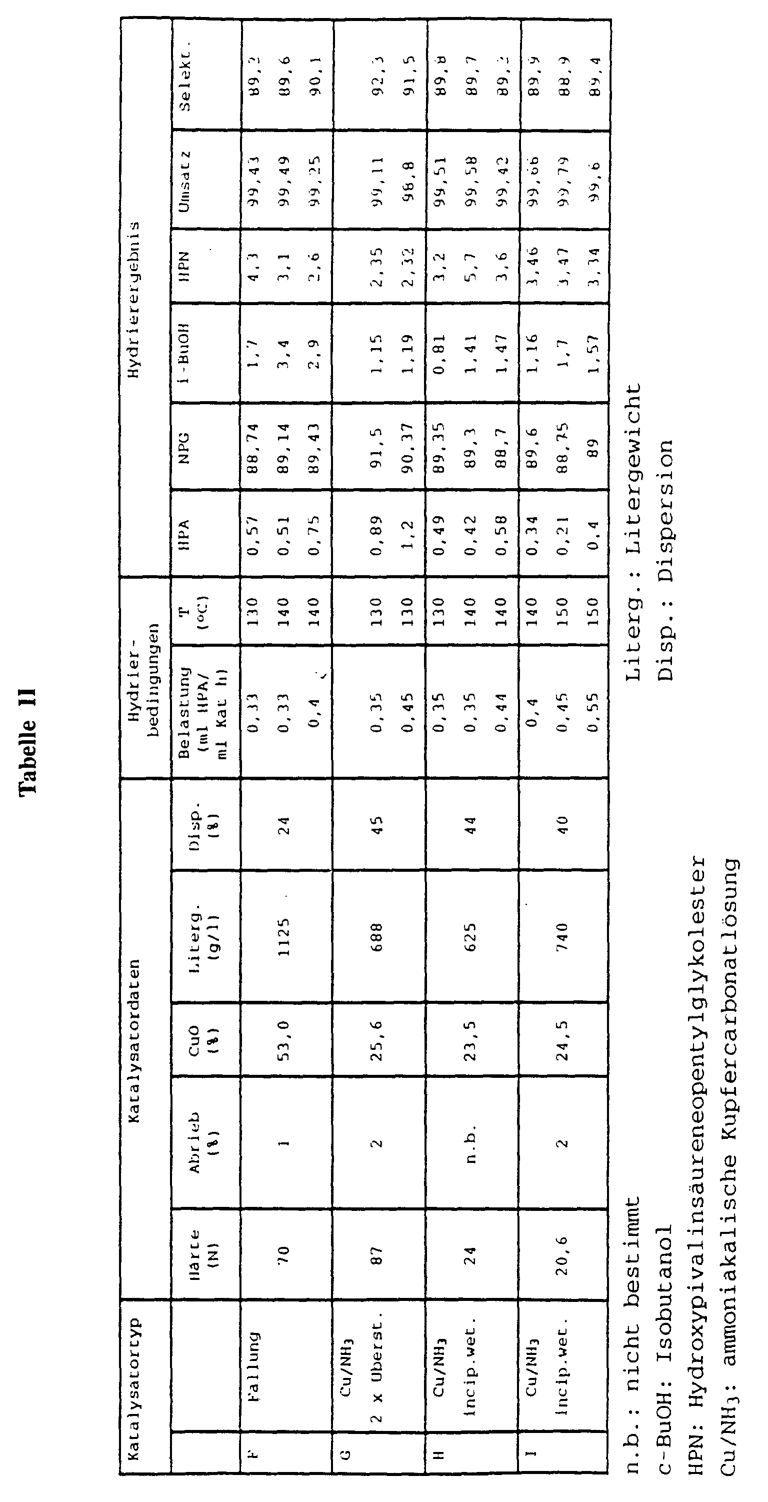

Der nach dem erfindungsgemäßen Verfahren hergestellte Katalysator G zeigt gegenüber Vergleichskatalysator F sowohl eine erhöhte Dispersion, wie auch verbesserte mechanische Eigenschaften bei hohem Umsatz und hoher Selektivität. Gegenüber den Katalysatoren H und I, die nach dem "incipient wetness"-Verfahren auf SiO₂-Basis hergestellt sind, zeichnet sich der auf einem SiO₂-Träger durch Tränkung in überstehender Lösung unter Verwendung leicht zersetzlicher Kupferverbindungen hergestellte Katalysator G durch verbesserte mechanische Eigenschaften, insbesondere eine erhöhte Härte aus.

### Beispiel 2.2: Hydrierung eines HPA/NPG-Gemisches zu Neopentylglykol (NPG)

Tabelle III zeigt die Ergebnisse der Hydrierung einer wäßrigen Lösung gleicher Gewichtsteile HPA und NPG, wobei HPA und NPG zusammen 76 Gew.-%, bezogen auf das Gesamtgewicht der Lösung, ergeben. Dabei betrug das Reaktorvolumen 1000 ml, das Katalysatorvolumen 700 ml. Der Reaktor wurde in Rieselfahrweise mit Produktrückführung (Kreislauf = 10,5 l/h) betrieben. Die angegebenen Katalysatorbelastungen beziehen sich auf HPA.

Der durch Tränkung in überstehender Lösung unter Verwendung leicht zersetzlicher Kupferverbindungen hergestellte Katalysator G zeigt gegenüber dem herkömmlich präparierten Katalysator F erhöhte Dispersion und verbesserte mechanische Eigenschaften bei höherem Umsatz und höherer Selektivität.

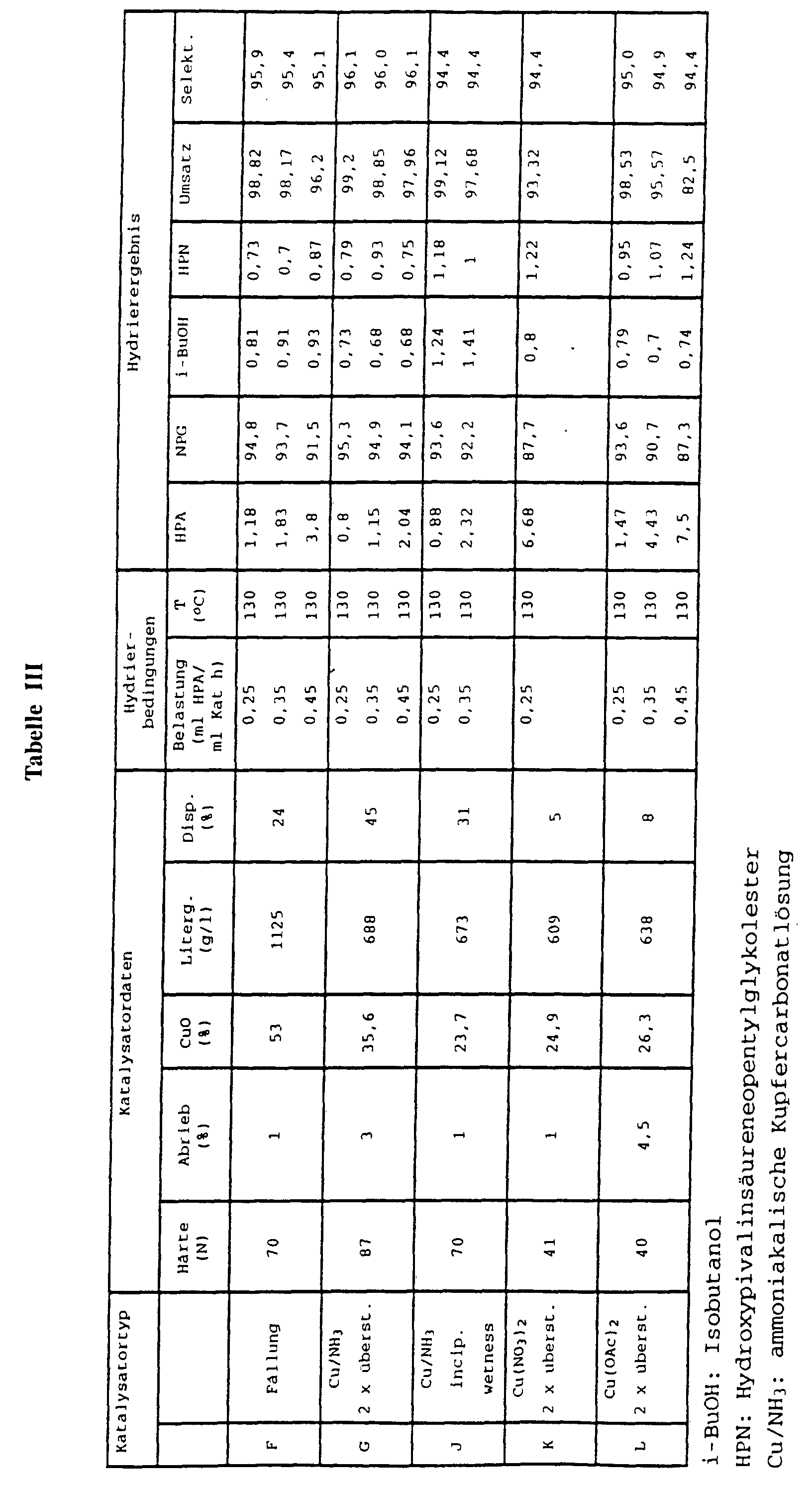

Im Vergleich der in überstehender Lösung getränkten Katalysatoren G und L zeigt der mit ammoniakalischer Kupfercarbonat-Lösung getränkte Katalysator G gegenüber dem mit ammoniakalischer Kupferacetat-Lösung getränkten Katalysator L deutliche Vorteile sowohl hinsichtlich der erreichten Dispersion und der erhaltenen mechanischen Stabilität, wie auch bezüglich des Umsatzes und der Selektivität bei hohen Belastungen.

### Beispiel 2.3: Hydrierung von i-Butanal (i-BA) und n-Butanal (n-BA) zu i-Butanol (i-Bol) und n-Butanol (n-Bol)

Hydriert wurde eine Mischung aus 12,5 Vol% i-Butanal und 87,5 Vol% n-Butanal. Dem Ausgangsgemisch wurden 20 ppm KOH zugegeben. Die Reaktion wurde in einem Volumen von 75 ml bei Sumpffahrweise im geraden Durchgang unter 50 bar Wasserstoffdruck durchgeführt. Die Ergebnisse sind in Tabelle IV dargestellt.

Verglichen wurden nur Katalysatoren auf SiO₂-Basis. Die Ausbeute ist bei den in überstehender Lösung unter Verwendung thermisch leicht zersetzbarer Kupferverbindungen getränkten Katalysatoren bei höheren Belastungen deutlich verbessert.

Bei dem in Tabelle IV angeführten Katalysator G' handelt es sich um den Katalysator G in reduziert/passivierter Form.

**Tabelle IV**

| **Bedingungen:** | | | **GC-Analytlk (GC-FI%)** | | | | | |
|---|---|---|---|---|---|---|---|---|
| Temp.: (°C) | Kat. | Bel.: | i-BA | n-BA | i-Bol | n-Bol | Sonst | Ausbeute |
| (Ausgangsmischung) | | | 12.40 | 87,10 | - | 0,47 | - | - |
| 150 | M | 0,33 | 0,22 | 1,27 | 11,40 | 86,60 | 0,51 | 98,00 |
| 150 | N | 0,33 | 0,00 | 0,00 | 13,09 | 85,84 | 1,07 | 98,93 |
| 140 | | 0,42 | 0,29 | 1,51 | 12,70 | 82.40 | 3,10 | 95,10 |
| 150 | | 0.33 | 0,00 | 0,00 | 11,70 | 87,40 | 0.90 | 99,10 |
| 150 | G | 0.42 | 0,00 | 0,00 | 13,30 | 86,40 | 0,30 | 99,70 |
| 150 | | 0,50 | 0,00 | 0,00 | 11,50 | 88,10 | 0,40 | 99,60 |
| 150 | | 0,33 | 0,00 | 0,00 | 12,16 | 87,45 | 0,39 | 99,61 |
| 140 | G' | 0,42 | 0,00 | 0,23 | 12,03 | 87,56 | 0,18 | 99,59 |
| 150 | | 0,50 | 0,00 | 0,19 | 13,00 | 86,53 | 0,28 | 99,53 |

| | | | | | | | | |
|---|---|---|---|---|---|---|---|---|
| Bel.: Belastung (ml Edukt/ml Kat·h) | | | | | | | | |

### Beispiel 2.4: Hydrierung von 2-Hydroxymethyl-2-methylbutanal zu 2-Methyl-2-ethyl-propandiol-1,3 (MEPD)

2-Hydroxymethyl-2-methylbutanal wurde in einer Lösung mit 35 Gew.-% Wasser und 22 Gew.-% Methanol, bezogen auf das Gesamtgewicht der Lösung, bei einem Reaktorvolumen von 75 ml in der Rieselfahrweise mit Produktrückführung (Kreislauf = 1,2 l/h) hydriert. Dabei herrschte ein Wasserstoffdruck von 30 bar und es wurden 55 ml Katalysator verwendet. Die Ergebnisse sind in Tabelle V dargestellt.

**Tabelle V**

| Katalysatortyp | Hydrierbedingungen | | Hydrierergebnisse | |
|---|---|---|---|---|
| | Belastung (ml Edukt/ ml Kat·h) | T (°C) | Umsatz (%) | Selektivität (%) |
| G | 0,2 | 130 | 100 | 96 |

### Beispiel 2.5: Hydrierung von (8,9),(3,4)-Diformyl-[5.2.1.0^{2,6}]tricyclodecan (TCD-Dialdehyd) zu (8,9),(3,4)-Bis(hydroxymethyl)-[5.2.1.0^{2,6}]tricyclodecan (TCD-Diol)

Eine Lösung von 50 Gew.-% TCD-Dialdehyd in THF wurde bei einem Reaktorvolumen von 75 ml und geradem Durchgang in Sumpffahrweise zu TCD-Diol hydriert. Die Gewichtsprozentangaben sind auf das Gesamtgewicht der Lösung bezogen. Die Ergebnisse sind in Tabelle VI angegeben.

**Tabelle VI**

| Katalysatortyp | Hydrierbedingungen | | | |
|---|---|---|---|---|
| | Belastung [ml Edukt/ ml Kat h] | T [°C] | Umsatz % | Selekt. % |
| G | 0,17 | 140 | 100 | 99 |
| | 0,29 | 140 | 99 | 90 |

### Beispiel 2.7: Hydrierung von 1-Formyl-1-hydroxymethyl-cyclopentan zu 1,1-Bis(hydroxymethyl)cyclopentan

Eine Lösung von 1-Formyl-1-hydroxymethyl-cylopentan mit 27 Gew.-% Wasser und 30 Gew.-% Methanol, bezogen auf das Gesamtgewicht der Lösung, wurde bei einem Reaktorvolumen von 75 ml bei geradem Durchgang in Sumpffahrweise hydriert. Dabei herrschte ein Wasserstoffdruck von 50 bar und es wurden 55 ml Katalysator eingesetzt. Die Ergebnisse sind in Tabelle VII dargestellt.

**Tabelle VII**

| Katalysatortyp | Hydrierbedingungen | | Hydrierergebnisse | |
|---|---|---|---|---|
| | Belastung (ml Edukt/ ml Kat·h) | T (°C) | Umsatz (%) | Selektivität (%) |
| G | 0,3 | 110 | 63 | 89 |
| | 0,3 | 140 | 93,3 | 82 |
| | 0,3 | 145 | 93,3 | 89 |
| | 0,22 | 145 | 99,3 | 91 |

### Beispiel 2.8: Hydrierung von 2-Ethylhexenal zu 2-Ethylhexanol

2-Ethylhexenal wurde in einem Reaktorvolumen von 75 ml in geradem Durchgang bei Sumpffahrweise hydriert. Der Wasserstoffdruck betrug dabei 50 bar. Das zu hydrierende 2-Ethylhexenal enthielt etwa 3 bis 4 ppm NaOH.

Die Katalysatoren G und O wurden dabei in reduziert-passivierter Form eingesetzt. Katalysator G' war ein in oxidischer Form eingesetzter und erst im Reaktor vorreduzierter Katalysator, der ansonsten Katalysator G entsprach. Die Ergebnisse sind in Tabelle VIII dargestellt.

Auch hier wird deutlich, daß die in überstehender Lösung getränkten Katalysatoren G und G' hohen Umsatz und hohe Selektivität auch bei hoher Belastung zeigen.

**Tabelle VIII**

| | | | GC-Analytik (GC-FI%) | | | | | |
|---|---|---|---|---|---|---|---|---|
| T (°C) | Kat. | Bel.: | Sonst. | enal | anal | anol | Umsatz | Selekt. |
| 150 | G | 0.20 | 3,45 | 1,37 | 0,88 | 94,30 | 98,63 | 95,61 |
| 150 | | 0,25 | 3,35 | 3,80 | 1,55 | 41,30 | 96,20 | 94,91 |
| 160 | | 0,25 | 3,85 | 1,49 | 0,76 | 93,90 | 98,51 | 95,32 |
| 160 | | 0,20 | 4,21 | 1,07 | 0,72 | 94,00 | 98,93 | 95,02 |
| 150 | G' | 0,25 | 3,25 | 0,60 | 0,00 | 96,15 | 99,40 | 96,73 |
| 150 | | 0,35 | 2,75 | 0,83 | 0,19 | 96,23 | 99,17 | 97,04 |
| 150 | | 0,50 | 2,82 | 0,85 | 0,63 | 95,70 | 99,15 | 96,52 |
| 150 | O | 0,20 | 3,15 | 0,26 | 4,66 | 91,93 | 99,74 | 92,17 |
| 160 | | 0,20 | 4,58 | 0,05 | 0,70 | 94,67 | 99,95 | 94,72 |
| 160 | | 0,25 | 3,80 | 0,30 | 4,10 | 91,80 | 99,70 | 92,08 |

| | | | | | | | | |
|---|---|---|---|---|---|---|---|---|
| enal: 2-Ethylhex-2-en-1-al | | | | | | | | |
| anal: 2-Ethylhexanal | | | | | | | | |
| anol: 2-Ethylhexanol | | | | | | | | |

### Beispiel 2.9: Hydrierung von Methyl-Ethylketon (MEK) zu sekundärem Butanol

Methyl-Ethylketon wurde in einem Reaktor mit einem Volumen von 75 ml zu sekundärem Butanol in Riesel-Kreislauffahrweise hydriert. Der Wasserstoffdruck betrug 40 bar. Es wurden 55 ml Katalysator verwendet. Die Ergebnisse sind in Tabelle IX dargestellt.

**Tabelle IX**

| Katalysatortyp | Hydrierbedingungen | | Hydrierergebnisse | |
|---|---|---|---|---|
| | Belastung (ml Edukt/ ml Kat·h) | T (°C) | Umsatz (%) | Selektivität (%) |
| G | 0,15 | 130 | 99,5 | 99,4 |
| | 0,25 | 130 | 97,87 | 99,5 |
| | 0,35 | 130 | 96,23 | 98,3 |
| | 0,45 | 130 | 96,75 | 98,5 |

## Patentansprüche

1. Verfahren zur Herstellung eines Katalysators, **dadurch gekennzeichnet, daß** Kupfer auf einen vorgefertigten SiO₂-haltigen Träger in An- oder Abwesenheit eines oder mehrerer der Elemente Magnesium, Barium, Zink oder Chrom, wobei der Katalysator 5 bis 50, vorzugsweise 5 bis 30 Gew.-% Kupfer, berechnet als CuO, und 50 bis 95, vorzugsweise 70 bis 95 Gew.-% Silicium, berechnet als SiO₂, jeweils bezogen auf das Gesamtgewicht des calcinierten Katalysators, enthält, durch Tränkung eines porösen Siliciumdioxid-Trägermaterials mit einer überstehenden wäßrigen Lösung einer thermisch leicht zersetzbaren Kupferverbindung aufgebracht wird und anschließend getrocknet und calciniert wird, wobei die Calcinierung bei Temperaturen von 200 bis 400, vorzugsweise von 250 bis 350 °C erfolgt.

2. Verfahren nach Anspruch 1, **dadurch gekennzeichnet, daß** die Summe der Katalysatorbestandteile Kupfer und Silicium und, soweit enthalten, Magnesium, Barium, Zink und Chrom 100 Gew.-% ergibt.

3. Verfahren nach Anspruch 2, **dadurch gekennzeichnet, daß** man einen Katalysator verwendet, der 5 bis 75 Gew.-% Kupfer, berechnet als CuO, und 95 bis 25 Gew.-% Si, berechnet als SiO₂, jeweils bezogen auf das Gesamtgewicht des calcinierten Katalysators, enthält.

4. Verfahren nach Anspruch 3, **dadurch gekennzeichnet, daß** man einen Katalysator verwendet, der außer Kupfer und Silicium eines oder mehrere der Elemente Magnesium, Barium, Zink oder Chrom enthält, wobei Magnesium in einer Menge von 0 bis 20 Gew.-%, berechnet als MgO, Barium in einer Menge von 0 bis 5 Gew.-%, berechnet als BaO, Zink in einer Menge von 0 bis 5 Gew.-%, berechnet als ZnO, und Chrom in einer Menge von 0 bis 5 Gew.-%, berechnet als Cr₂O₃, jeweils bezogen auf das Gesamtgewicht des calcinierten Katalysators, enthalten ist, mit der Maßgabe, daß die Summe der Katalysatorbestandteile Kupfer, Silicium und, soweit enthalten, Magnesium, Barium, Zink und Chrom 100 Gew.-% ergibt.

5. Katalysator, erhältlich gemäß einem Verfahren nach einem der vorhergegangenen Ansprüche.

6. Verwendung eines Katalysators gemäß Anspruch 5 zur Herstellung von Alkoholen aus entsprechenden Carbonylverbindungen.

7. Verfahren zur Herstellung von Alkoholen durch katalytische Hydrierung der entsprechenden Carbonylverbindungen mit einem Katalysator, **dadurch gekennzeichnet, daß** man einen Katalysator nach Anspruch 5 einsetzt und daß man die Carbonylverbindungen bei erhöhter Temperatur und erhöhtem Druck, vorzugsweise bei einer Temperatur von 60 bis 200 °C und bei einem Druck von 1 bis 150 bar, in flüssiger Phase hydriert.

8. Verfahren nach Anspruch 7, **dadurch gekennzeichnet, daß** man bei der Hydrierung einen pH-Wert der flüssigen Phase von 4 bis 13 einstellt.

9. Verfahren nach Anspruch 7 oder 8, **dadurch gekennzeichnet, daß** man als Carbonylverbindungen C₂- bis C₂₀- Hydroxycarbonylverbindungen einsetzt und daraus die entsprechenden Diole erzeugt.

10. Verfahren nach Anspruch 9, **dadurch gekennzeichnet, daß** man als Carbonylverbindungen Hydroxypropionaldehyde der allgemeinen Formel I 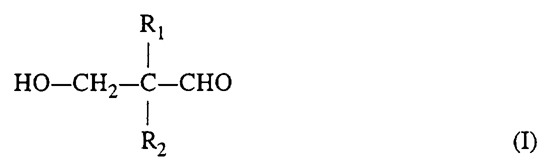 einsetzt und daraus 1,3-Propandiole der allgemeinen Formel II 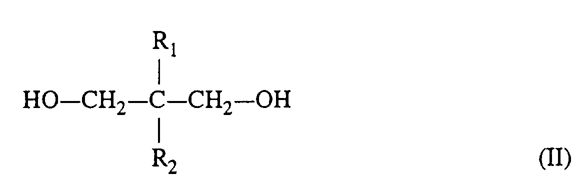 herstellt, worin die Reste R¹ und R² gleich oder verschieden sind und jeweils für Wasserstoff, eine C₁- bis C₂₄-Alkyl-, eine C₆- bis C₂₀-Aryl-und/oder eine C₇- bis C₁₂-Aralkylgruppe stehen oder beide Reste R¹ und R² gemeinsam mit den benachbarten Kohlenstoffatomen einen 5 bis 10-gliedrigen alicyclischen Ring bilden.

11. Verfahren nach einem der Ansprüche 7 bis 9, **dadurch gekennzeichnet, daß** man nacheinander
- Isobutyraldehyd und Formaldehyd wäßrig in Gegenwart von Trialkylamin umsetzt,
- das nicht umgesetzte Isobutyraldehyd aus der wäßrigen Lösung, vorzugsweise durch Destillation, abtrennt,
- die wäßrige Lösung der Umsetzung, die Hydroxypivalinaldehyd, Trialkylamin und andere Verunreinigungen und Nebenprodukte enthält, mit Wasserstoff in Gegenwart des Kupferkatalysators hydriert, und
- aus der sich ergebenden wäßrigen Phase Neopentylglykol, vorzugsweise durch Destillation, gewinnt.

12. Verfahren nach einem der Ansprüche 7 bis 9, **dadurch gekennzeichnet, daß** man als Carbonylverbindungen geradkettige oder verzweigte aliphatische Aldehyde oder Ketone mit 1 bis 24 Kohlenstoffatomen oder alicyclische C₃- bis C₁₂-Ketone einsetzt und hieraus die entsprechenden Alkohole erzeugt.

13. Verfahren nach einem der Ansprüche 7 bis 9, **dadurch gekennzeichnet, daß** man als Carbonylverbindungen geradkettige oder verzweigte aliphatische C₃- bis C₁₂-α,β-ungesättigte Carbonylverbindungen einsetzt und hieraus die entsprechenden gesättigten Alkohole erzeugt.

## Claims

1. A process for the preparation of a catalyst, wherein copper is applied to a prepared SiO₂-containing support in the presence or absence of one or more of the elements magnesium, barium, zinc or chromium, the catalyst containing from 5 to 50, preferably from 5 to 30, % by weight of copper, calculated as CuO, and from 50 to 95, preferably from 70 to 95, % by weight of silicon, calculated as SiO₂, based in each case on the total weight of the calcined catalyst, by impregnating a porous silica support material with a supernatant aqueous solution of a thermally readily decomposable copper compound and then drying and calcination are effected, the calcination being effected at from 200 to 400°C, preferably from 250 to 350°C.

2. A process as claimed in claim 1, wherein the sum of the catalyst components copper and silicon and, if present, magnesium, barium, zinc and chromium is 100% by weight.

3. A process as claimed in claim 2, wherein the catalyst used contains from 5 to 75% by weight, calculated as CuO, of copper and from 95 to 25% by weight, calculated as SiO₂, of Si, based in each case on the total weight of the calcined catalyst.

4. A process as claimed in claim 3, wherein the catalyst used contains one or more of the elements magnesium, barium, zinc and chromium in addition to copper and silicon, magnesium being present in an amount of from 0 to 20% by weight, calculated as MgO, barium in an amount of from 0 to 5% by weight, calculated as BaO, zinc in an amount of from 0 to 5% by weight, calculated as ZnO, and chromium in an amount of from 0 to 5% by weight, calculated as Cr₂O₃, based in each case on the total weight of the calcined catalyst, with the proviso that the sum of the catalyst components copper, silicon and, if present, magnesium, barium, zinc and chromium is 100% by weight.

5. A catalyst obtainable by a process as claimed in any of the preceding claims.

6. The use of a catalyst as claimed in claim 5 for the preparation of an alcohol from the corresponding carbonyl compound.

7. A process for the preparation of an alcohol by the catalytic hydrogenation of the corresponding carbonyl compound using a catalyst, wherein a catalyst as claimed in claim 5 is used and wherein the carbonyl compounds are hydrogenated at elevated temperature and superatmospheric pressure, preferably at from 60 to 200°C and at from 1 to 150 bar, in the liquid phase.

8. A process as claimed in claim 7, wherein a pH of the liquid phase of from 4 to 13 is established during the hydrogenation.

9. A process as claimed in claim 7 or 8, wherein a C₂-C₂₀-hydroxycarbonyl compound is used as the carbonyl compound and is converted into the corresponding diol.

10. A process as claimed in claim 9, wherein a hydroxypropionaldehyde of the formula I 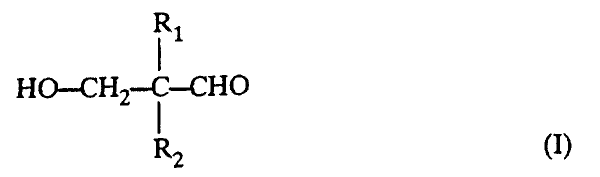 is used as the carbonyl compound and is converted into a 1,3-propanediol of the formula II 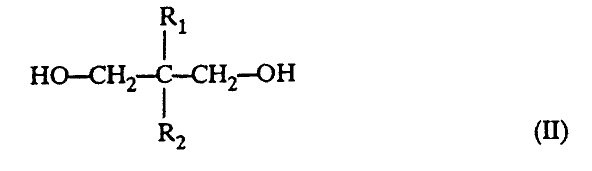 where R¹ and R² are identical or different and are each hydrogen, C₁-C₂₄-alkyl or C₆-C₂₀-aryl or C₇-C₁₂-aralkyl or the two radicals R¹ and R², together with the neighboring carbon atoms, form a 5-membered to 10-membered alicyclic ring.

11. A process as claimed in any of claims 7 to 9, wherein the following are carried out in succession:
- isobutyraldehyde and formaldehyde are reacted in an aqueous medium in the presence of a trialkylamine,
- the unconverted isobutyraldehyde is separated off from the aqueous solution, preferably by distillation,
- the aqueous reaction solution, which contains hydroxypivalaldehyde, trialkylamine and other impurities and byproducts is hydrogenated with hydrogen in the presence of a copper catalyst and
- neopentylglycol is recovered from the resulting aqueous phase, preferably by distillation.

12. A process as claimed in any of claims 7 to 9, wherein a straight-chain or branched aliphatic aldehyde or ketone of 1 to 24 carbon atoms or an alicyclic C₃-C₁₂-ketone is used as the carbonyl compound and is converted into the corresponding alcohol.

13. A process as claimed in any of claims 7 to 9, wherein a straight-chain or branched aliphatic C₃-C₁₂-α,β-unsaturated carbonyl compound is used as the carbonyl compound and is converted into the corresponding saturated alcohol.

## Revendications

1. Procédé de fabrication d'un catalyseur, **caractérisé en ce que** du cuivre est appliqué sur un support contenant du SiO₂, préfabriqué, en absence ou en présence d'un ou plusieurs des éléments que sont le magnésium, baryum, zinc ou chrome, le catalyseur contenant de 5 à 50 %, de préférence 5 à 30 % en poids de cuivre sous forme de CuO, ou de 50 à 95 %, de préférence de 70 à 95 % de silicium, calculé sous forme de SiO₂, chaque fois en se référant au poids global du catalyseur calciné, l'application se faisant par imbibition d'un matériau support à base de dioxyde de silicium poreux, par une solution aqueuse, placée sur lui, d'une combinaison de cuivre aisément décomposable thermiquement, puis est séchée et calcinée, la calcination se faisant à des températures de 200 à 400, de préférence de 250 à 350 °C.

2. Procédé selon la revendication 1, **caractérisé en ce que** la somme des constituants du catalyseur que sont le cuivre et le silicium et, dans la mesure où il y en a, le magnésium, le baryum, le zinc et le chrome, font 100 % en poids.

3. Procédé selon la revendication 2, **caractérisé en ce qu'**on utilise un catalyseur qui contient de 5 à 75 % en poids de cuivre, calculé sous forme de CuO, et de 95 à 25 % en poids de Si, calculé sous forme de SiO₂, chaque fois en se référant au poids global du catalyseur calciné.

4. Procédé selon la revendication 3, **caractérisé en ce qu'**on utilise un catalyseur qui contient, outre du cuivre et du silicium, un ou plusieurs des éléments qui sont le magnésium, baryum, zinc ou chrome, le magnésium étant contenu en une quantité de 0 à 20 % en poids, calculé sous forme de MgO, le baryum étant contenu en une quantité de 0 à 5 % en poids, calculé sous forme de BaO, le zinc étant contenu en une quantité de 0 à 5 %, calculé sous forme de ZnO, et le chrome étant contenu en une quantité de 0 à 5 % en poids, calculé sous forme de Cr₂O₃, chaque fois en se référant au poids global du catalyseur calciné, avec l'indication que la somme des constituants du catalyseur, que sont le cuivre et le silicium, et, dans la mesure où il y en a, le magnésium, le baryum, le zinc et le chrome, font 100 % en poids.

5. Catalyseur, obtenu suivant un procédé selon l'une des revendications précédentes.

6. Utilisation d'un catalyseur selon la revendication 5, pour la fabrication d'alcools à partir de combinaisons carbonyle correspondantes.

7. Procédé de fabrication d'alcools par hydrogénation catalytique des combinaisons carbonyle correspondantes avec un catalyseur, **caractérisé en ce qu'**on utilise un catalyseur selon la revendication 5 et que l'on hydrogène les combinaisons carbonyle sous haute température et haute pression, de préférence à une température de 60 à 200 °C et à une pression de 1 à 150 bar, en phase liquide.

8. Procédé selon la revendication 7, **caractérisé en ce que**, lors de l'hydrogénation, on règle une valeur de pH de la phase liquide, de 4 à 13.

9. Procédé selon la revendication 7 ou 8, **caractérisé en ce que** l'on utilise comme combinaisons carbonyles des combinaisons hydroxycarbonyle en C₂ à C₂₀ et qu'on génère, à partir de celles-ci, les diols correspondants.

10. Procédé selon la revendication 9, **caractérisé en ce qu'**on utilise comme combinaisons carbonyle des hydroxypropionaldéhydes de formule générale I 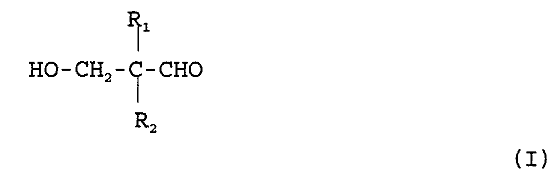 et, à partir de cela, on prépare des 1,3 propandiols de formule générale II 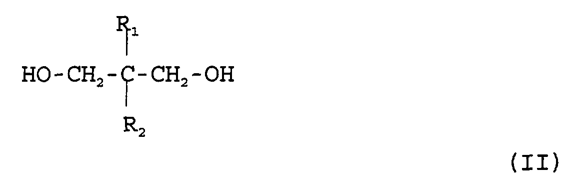 dans laquelle les résidus R¹ et R² sont identiques ou différents et chaque fois correspondent à de l'hydrogène, un alkyl en C₁ à C₂₄, un aryl en C₆ à C₂₀ et/ou un groupe aralkyl en C₇ à C₁₂, ou les deux résidus R₁ et R₂ forment conjointement, avec les atomes de carbones voisins, un anneau alicyclique de 5 à 10 maillons.

11. Procédé selon l'une des revendications 7 à 9, **caractérisé en ce que**, l'un après l'autre,
- on convertit l'isobutyraldéhyde et le formaldéhyde de façon aqueuse, en présence de trialkylamine,
- on sépare l'isobutyraldéhyde non converti pour l'extraire de la solution aqueuse, de préférence par distillation,
- on hydrogène la solution aqueuse de la conversion, qui contient de l'hydroxypivalinaldéhyde, de la trialkylamine et d'autres impuretés et produits annexes, avec de l'hydrogène, en présence du catalyseur à base de cuivre, et
- à partir de la phase aqueuse résultante, on obtient du néopentylglycol, de préférence par distillation.

12. Procédé selon l'une des revendications 7 à 9, **caractérisé en ce qu'**on utilise, à titre de combinaisons carbonyle, des aldéhydes aliphatiques à chaînes droites ou ramifiées, ou des cétones comportant de 1 à 24 atomes de carbone ou des cétones alicycliques en C₃ à C₁₂ et on produit à partir de cela les alcools correspondants.

13. Procédé selon l'une des revendications 7 et 9, **caractérisé en ce que** l'on utilise à titre de combinaisons carbonyle des combinaisons carbonyle en C₃ à C₁₂ α, β insaturées, aliphatiques à chaînes droites ou ramifiées, et on produit à partir de cela les alcools saturés correspondants.
